(19) Europäisches Patentamt
European Patent Office
Office européen des brevets

(11) Veröffentlichungsnummer: **0 508 279 A1**

(12) **EUROPÄISCHE PATENTANMELDUNG**

(21) Anmeldenummer: **92105526.5**

(51) Int. Cl.[5]: **C07C 263/16**

(22) Anmeldetag: **31.03.92**

(30) Priorität: **12.04.91 DE 4111905**

(43) Veröffentlichungstag der Anmeldung:
**14.10.92 Patentblatt 92/42**

(84) Benannte Vertragsstaaten:
**BE CH DE FR GB IT LI NL**

(71) Anmelder: **BAYER AG**
**W-5090 Leverkusen 1 Bayerwerk(DE)**

(72) Erfinder: **Schubart, Rüdiger, Dr.**
**An der Engelsfuhr 27**
**W-5060 Bergisch Gladbach 2(DE)**

(54) **Verfahren zur Herstellung von beta-Halogen-tert.-alkylisocyanaten.**

(57) Die Erfindung betrifft ein neues Verfahren zur Herstellung von bekannten ß-Halogen-tert.-alkylisocyanaten der Formel

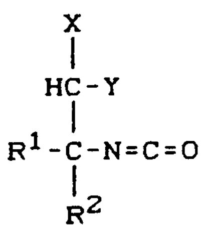

(I)

in welcher
X für Chlor steht,
Y für Wasserstoff oder Chlor steht,
$R^1$ für jeweils geradkettiges oder verzweigtes Alkyl oder Halogenalkyl steht und
$R^2$ für jeweils geradkettiges oder verzweigtes Alkyl oder Halogenalkyl oder für gegebenenfalls durch Halogen und/oder Trifluormethyl substituiertes Phenyl steht,
wobei man tert.-Alkylisocyanate der Formel (II)

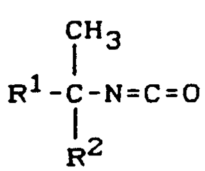

(II)

mit Chlor gegebenenfalls unter Belichtung oder in Gegenwart von Katalysatoren in einer geeigneten Apparatur umsetzt.

EP 0 508 279 A1

Die Erfindung betrifft ein neues Verfahren zur Herstellung von bekannten ß-Halogen-tert.-alkylisocyanaten, die als Zwischenprodukte für die Synthese von Kautschukhilfsmitteln oder von biologisch aktiven Verbindungen, wie beispielsweise Herbiziden (vgl. z.B. EP-A 294 666), eingesetzt werden können.

Es ist bereits bekannt, daß man ß-Monochlor-tert,-alkylisocyanate erhält, wenn man ß-Hydroxy-tert,-alkylamine zunächst mit Thionylchlorid hydrochloriert und dann das entsprechende Chloramin-hydrochlorid mit Phosgen in das Isocyanat überführt (vgl. DE-OS 2 045 906).

Nachteilig an diesem Verfahren sind die schlechten Ausbeuten an den chlorierten tert.-Alkylisocyanaten.

Es ist ferner bereits bekannt, Methylisocyanat mit Chlor unter Belichtung umzusetzen (vgl. Houben-Weyl Band E4, Seite 1171). Auf diese Weise erhält man jedoch das mehrfach chlorierte Chlorcarbonyl-isocyaniddichlorid.

Es wurde nun gefunden, daß man ß-Halogen-tert.-alkylisocyanate der Formel (I)

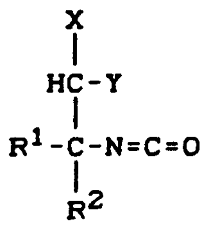

(I)

in welcher

- X für Chlor steht,
- Y für Wasserstoff oder Chlor steht,
- $R^1$ für jeweils geradkettiges oder verzweigtes Alkyl oder Halogenalkyl steht und
- $R^2$ für jeweils geradkettiges oder verzweigtes Alkyl oder Halogenalkyl oder für gegebenenfalls durch Halogen und/oder Trifluormethyl substituiertes Phenyl steht,

in guten Ausbeuten und hoher Reinheit erhält, wenn man tert.-Alkylisocyanate der Formel (II)

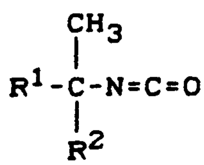

(II)

in welcher

$R^1$ und $R^2$ die oben angegebene Bedeutung haben,

mit elementarem Chlor, gegebenenfalls unter Belichtung oder in Gegenwart von Katalysatoren in einer erfindungsgemäßen, geeigneten Apparatur (siehe hierzu beispielsweise DE 27 16 896), umsetzt, wofür 2 Verfahrensvarianten nämlich eine Sumpfhalogenierung und eine kontinuierliche Halogenierung zur Verfügung stehen.

Es ist ausgesprochen überraschend, daß die Chlorierung in guter Ausbeute die gewünschten ß-Halogen-tert.-alkylisocyanate der Formel (I) ergibt. Da bei der Reaktion HCl frei wird, mußte vielmehr erwartet werden, daß sich HCl an das Isocyanat anlagert (vgl. Houben Weyl, Band E4, Seite 57-58) und dadurch die Ausbeute deutlich verringert wird. Ferner mußte bei der Umsetzung von Isocyanaten mit Halogen unter Belichtung mit Fragmentierungen (vgl. Houben Weyl 4/5b Photochemie I und II, 891-892) oder mit Abspaltung des tert.-Alkylrestes in Form von tert.-Alkylchlorid (vgl. Houben Weyl Bd. E4 S. 63) gerechnet werden.

Mit Hilfe des erfindungsgemäßen Verfahrens erhält man bevorzugt Verbindungen der Formel (I), in welcher $R^1$ für Methyl, Ethyl, n- oder iso-Propyl, n-, iso-, sec.- oder tert.-Butyl steht, $R^2$ für Methyl, Ethyl, n-oder iso-Propyl, n-, iso-, sec,- oder tert.-Butyl oder für gegebenenfalls einfach bis dreifach gleich oder verschieden durch Fluor, Chlor, Brom und/oder Trifluormethyl substituiertes Phenyl steht, besonders bevorzugt stehen $R^1$ für Methyl oder Ethyl und $R^2$ für Methyl, Ethyl oder Phenyl, insbesondere für Methyl.

Ganz besonders bevorzugt lassen sich nach dem erfindungsgemäßen Verfahren die Verbindungen der Formel (I) herstellen, in denen X für Chlor und Y für Wasserstoff stehen, d.h. ß-monochlorierte tert.-Alkylisocyanate.

Das erfindungsgemäße Verfahren läßt sich bei Verwendung von tert.-Butylisocyanat durch folgendes

Formelschema darstellen:

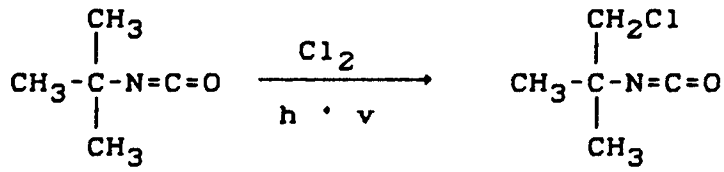


Das erfindungsgemäße Verfahren kann auch in Gegenwart von Verdünnungsmitteln durchgeführt werden. Man kann alle für solche Halogenierungsreaktionen üblichen Verdünnungsmittel wie beispielsweise Dichlormethan, Chloroform oder Tetrachlormethan einsetzen. Bevorzugt verwendet man Tetrachlormethan.

Vorzugsweise wird das erfindungsgemäße Verfahren ohne Zusatz von Verdünnungsmitteln durchgeführt.

Die direkte Halogenierung von tert.-Alkylisocyanaten der Formel (II) kann gegebenenfalls unter Belichtung oder durch Zugabe von geeigneten Katalysatoren durchgeführt werden.

Die Bestrahlung wird beispielsweise mit einem wassergekühlten Quecksilber-Hochdruckbrenner durchgeführt, wobei die Halogenierungslampe sowohl als Tauchlampe eingesetzt, als auch von außen angebracht werden kann, Bei der Anbringung der Lampe ist darauf zu achten, daß möglichst viel Licht in die Halogenierungszone gelangt. Alle für solche Halogenierungen üblichen Quecksilber-Hochdruckbrenner lassen sich für das erfindungsgemäße Verfahren einsetzen. Natürlich sind auch andere für solche Halogenierungen geeignete Lampen einsetzbar.

Als Katalysatoren verwendet man vorzugsweise Peroxide, wie beispielsweise Cumylperoxid oder Benzoylperoxid, oder Azoverbindungen wie beispielsweise Azoisobuttersäurenitril (AIBN).

Die Reaktionstemperaturen können bei der Durchführung des erfindungsgemäßen Verfahrens in einem größeren Bereich variiert werden. Im allgemeinen arbeitet man bei Temperaturen zwischen 40 °C und 120 °C, bevorzugt zwischen 60 °C und 110 °C, besonders bevorzugt zwischen 85 °C und 100 °C. Insbesondere arbeitet man bei Temperaturen, die im Bereich der Siedetemperatur des zu chlorierenden tert.-Alkylisocyanats bzw. des Verdünnungsmittels liegen.

Das erfindungsgemäße Verfahren kann in zwei unterschiedlichen Varianten durchgeführt werden und zwar als sogenannte Sumpfhalogenierung (Variante 1) oder in Form einer kontinuierlichen Halogenierung (Variante 2).

Zur Durchführung von Variante 1 des Verfahrens (Sumpfhalogenierung) setzt man für den Fall, daß die monohalogenierten Verbindungen der Formel (I) (d.h. X = Chlor und Y = Wasserstoff) erhalten werden sollen, pro Mol tert.-Alkylisocyanat der Formel (II) im allgemeinen äquivalente Mengen oder einen geringfügigen Unterschuß an Halogen ein. Für den Fall, daß die dihalogenierten Verbindungen der Formel (I) hergestellt werden sollen, setzt man pro Mol tert.-Alkylisocyanat der Formel (II) einen Überschuß, bevorzugt bis 2,5 Mol, besonders bevorzugt bis 2,1 Mol an Halogen ein.

In einer für Variante 2 geeigneten Apparatur, die beispielsweise aus einem Destillationskolben, einer Füllkörperkolonne, einem Kühler und der Chlorierungseinrichtung besteht, wird zunächst Edukt verdampft. Dieses gelangt durch die Kolonne zum Kühler und kondensiert dort. Das Kondensierte Edukt kann nun ganz oder teilweise durch die Chlorierungszone, die gleichzeitig belichtet und mit Chlor beschickt wird, geführt werden. Das in dieser Chlorierungszone entstandene Reaktionsgemisch, bestehend aus Edukt und Produkt der Formel(I) wird nun der Trennkolonne zwecks Trennung in die Komponenten zugeführt. Eine entsprechend geeignete Apparatur ist zum Beispiel in der DE 27 16 896 beschrieben. Prinzipiell sind aber auch andere Apparaturen für diesen Kreislaufprozeß einsetzbar.

Zur Durchführung von Variante 2 des Verfahrens (kontinuierliche Halogenierung) setzt man für den Fall, daß die monohalogenierten Verbindungen der Formel (I) erhalten werden sollen, pro Mol tert.-Alkylisocyanat der Formel (II) im allgemeinen äquivalente Mengen bzw. einen geringfügigen Überschuß an Halogen ein. Für den Fall, daß die dihalogenierten Verbindungen der Formel (I) hergestellt werden sollen, setzt man pro Mol tert.-Alkylisocyanat im allgemeinen äquivalente Mengen oder einen Überschuß, bevorzugt bis 2,25 Mol bzw, besonders bevorzugt bis 2,05 Mol an Halogen ein. Am zweckmäßigsten setzt man bei der kontinuierlichen Halogenierung zur Herstellung der dihalogenierten Verbindungen einen leichten Überschuß an Halogen ein.

Falls das Verfahren in Gegenwart von Katalysatoren durchgeführt wird, setzt man pro Mol tert.-Alkylisocyanat der Formel (II) im allgemeinen 0,001 bis 1,5 Mol-%, bevorzugt 0,02 bis 1 Mol-% und besonders bevorzugt 0,1 bis 0,5 Mol-% an Katalysator zu.

Bei der Sumpfhalogenierung geht man so vor, daß unverdünntes Chlor in siedendes unverdünntes oder gegebenenfalls verdünntes tert.-Alkylisocyanat der Formel (II) eingeleitet wird,

Man kann die Halogenierungsreaktion bis zu fast vollständigem Umsatz, in der Regel bis zu 95 % laufen lassen, oder man unterbricht die Halogenierungsreaktion bereits zu einem früheren Umsatzzeitpunkt, z.B. bei etwa 60 % Umsatz. Umsatz wird hier definiert als 100 % Edukt minus noch vorhandenes Edukt. Das Reaktionsgemisch wird dann fraktioniert, um nicht halogeniertes Edukt zurückzugewinnen, welches erneut der Reaktion zugeführt wird.

Bei der Sumpfhalogenierung wird die Reaktion im allgemeinen bei einem Umsatz von 40 bis 80 %, bevorzugt 45-65 %, besonders bevorzugt 50-55 % unterbrochen.

Der Vorteil dieser Verfahrensweise liegt darin, daß der Anteil der Nebenprodukte und damit die Menge des Materialverlustes (d.h. Verlust an Edukt der Formel (II)) deutlich geringer wird.

Auf diese Weise läßt sich die Ausbeute an ß-Halogen-tert.-alkylisocyanat aus einer bestimmten Menge tert.-Alkylisocyanat deutlich verbessern.

Bei der kontinuierlichen Verfahrensweise wird tert.-Butylisocyanat und Chlor getrennt einer Reaktionszone, vorzugsweise unter Belichtung oder Zugabe eines Katalysators, zugeführt und das Reaktionsgemisch, welches Edukt der Formel (II) und Produkt der Formel (I) enthält, wird ständig der Halogenierungszone entnommen. Dieses Gemisch wird einer Kolonne zur Fraktionierung zugeführt und nicht halogeniertes Isocyanat, d.h. Edukt der Formel (11), sofort abgetrennt und mit frischem tert.-Alkylisocyanat der Halogenierungszone erneut zugeführt. Das halogenierte tert.-Alkylisocyanat, d.h. Verbindungen der Formel (I), wird am Boden der Apparatur abgesondert und im Falle genügender Reinheit direkt für weitere Umsetzungen verwendet oder ständig der Apparatur entnommen und einer Feinfraktionierung unterworfen.

Auf diese Weise entsteht ein kontinuierlich arbeitender Prozeß, bei dem ständig Reaktionsgemisch entnommen und frisches Edukt sowie Chlor zugeführt werden.

Die Vermischung des Halogens in der gegebenenfalls völlig gefluteten Reaktionszone kann z.B durch Rühren, durch die Strömung der Reaktionspartner bei der Zugabe in einem Venturi-Düsenrohr oder eine Kombination dieser Möglichkeiten sowie mit Hilfe von anderen möglichen geeigneten Vorrichtungen erfolgen.

Auf diese Weise erhält man gezielt mono- bzw. dihalogenierte Verbindungen der Formel (I) in hoher Ausbeute und Reinheit und hält die Bildung von Nebenprodukten gering. Mit einer relativ kleinen Apparatur lassen sich durch die kontinuierliche Verfahrensweise pro Zeiteinheit somit große Mengen an gewünschtem Produkt herstellen.

Bei der kontinuierlichen Reaktionsführung wird die Chlorierung des durch die Chlorierungszone laufenden tert.-Butylisocyanats im allgemeinen bis zu einem Umsatz zwischen 0,1 und 30 %, vorzugsweise zwischen 2 und 20 %, besonders bevorzugt zwischen 5 und 15 % durchgeführt und das entstandene Produkt laufend abgetrennt, so daß insgesamt Chlor und tert.-Alkylisocyanat der Apparatur zugeführt und chloriertes tert.-Alkylisocyanat praktisch ohne Edukt der Apparatur entnommen werden.

Die Reaktion kann sowohl bei der Sumpfhalogenierung als auch bei der kontinuierlichen Halogenierung unterbrochen und der Prozeß nach einiger Zeit fortgesetzt werden. Das Reaktionsgemisch kann auch zwischengelagert und erst nach einiger Zeit weiterverarbeitet werden.

Prinzipiell sind beide Verfahrensvarianten auch zur Bromierung von tert.-Alkylisocyanaten anwendbar.

Das erfindungsgemäße Verfahren wird durch folgende Beispiele veranschaulicht.

Beispiel 1

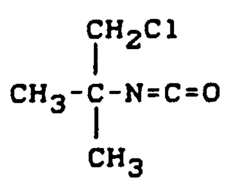

Sumpfhalogenierung

812 g tert.-Butylisocyanat werden vorgelegt und bei 85 bis 100 °C unter Belichtung mit einer Hg-Hochdruckbrenner-Tauchlampe (wassergekühlt) unter Rühren durch direktes Einleiten von unverdünntem Chlor chloriert. Es werden Proben entnommen und der Verlauf der Chlorierung mittels Gaschromatographie (GC) verfolgt. Nach einem Umsatz von 95 % wird die Reaktion abgebrochen. Man erhält 1225 g

Rohprodukt, das über eine 20 cm Füllkörperkolonne (mit 3 mm Raschigglasringen) fraktioniert wird.

Die Zusammensetzung des Chlorierungsgemisches ist unmittelbar nach beendeter Chlorierung folgende (in Flächen-%):

| | | | |
|---|---|---|---|
| 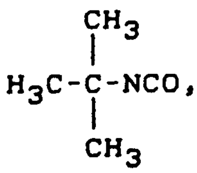 | 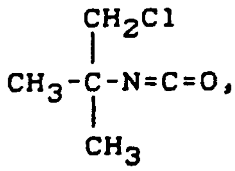 | 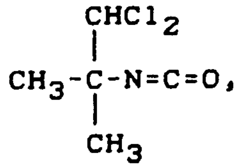 | 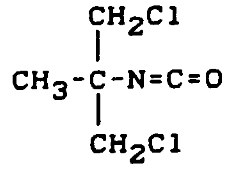 |
| 5,6 % | 58,5 % | 17,6 % | 13,9 % |

Man erhält:

1. Fraktion

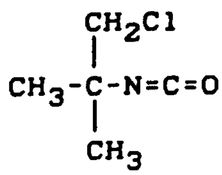

gewünschtes Produkt
Ausbeute: 627 g (60,7 % der Theorie)
bezogen auf eingesetztes Isocyanat minus 5 %, da nur bis 95 % Umsatz chloriert wurde.
Siedepunkt: 44,5 ° C/15 mbar
Reinheit: 98,5 % (GC)

2. Fraktion:

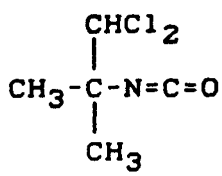

Ausbeute: 177,5 g
Siedepunkt: 61,5-62 ° C/19 mbar
Reinheit: 96,8 % (GC)

3. Fraktion:

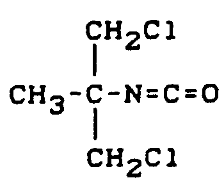

Ausbeute: 80,5 g
Siedepunkt: 65 ° C/18 mbar
Reinheit: 96,3 % (GC)

<u>Beispiel 2</u>

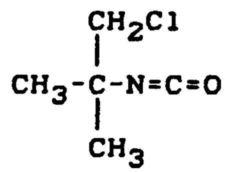

Kontinuierliche Verfahrensweise:

In einer Apparatur, die für solch eine kontinuierliche Chlorierung geeignet ist, werden mindestens 800 g tert.-Butylisocyanat vorgelegt und wie oben beschrieben durch Teilchlorierung des im Kreislauf befindlichen Eduktes langsam chloriert. Wenn der Umsatz ungeführ 95 % erreicht hat, kann die Reaktion abgebrochen werden, oder es kann tert.-Butylisocyanat und Chlor im Verhältnis 1:1,05 ständig der Apparatur bzw. vor der Chlorierungszone zugeführt und chloriertes Produkt nahezu völlig ohne Edukt der Apparatur entnommen werden.

Aus 800 g tert.-Butylisocyanat werden bei einem Umsatz von 95 % bis zu 1027 g Monochlor-tert.-butylisocyanat (Siedepunkt 44,5° C bei 15 mbar, $n_D^{20}$ 1,4355) mit einer Reinheit bis 98,2 % gewonnen, wobei die Rohzusammensetzung aus folgenden Produkten bestand (in Flächen-%):

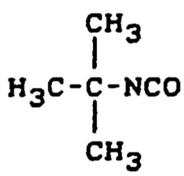 , 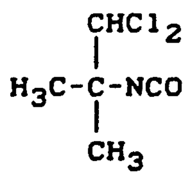 , 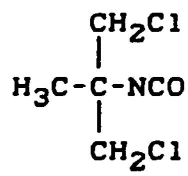 , 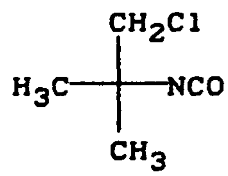

4,7 % 2,2 % 2,4 % 88,6 %

Dies entspricht einer theoretischen Ausbeute von 89,7 % Monochlor-tert.-butylisocyanat, wobei 4,7 % tert.-Butylisocyanat zurückgewonnen und somit vom Einsatz zur Berechnung abgezogen wurden.

**Patentansprüche**

**1.** Verfahren zur Herstellung von ß-Halogen-tert.-alkylisocyanaten der Formel (I)

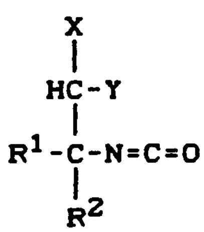 (I)

in welcher

X für Chlor steht,

Y für Wasserstoff oder Chlor steht,

$R^1$ für jeweils geradkettiges oder verzweigtes Alkyl oder Halogenalkyl steht und

$R^2$ für jeweils geradkettiges oder verzweigtes Alkyl oder Halogenalkyl oder für gegebenenfalls durch Halogen und/oder Trifluormethyl substituiertes Phenyl steht,

dadurch gekennzeichnet, daß man tert.-Alkylisocyanate der Formel (II)

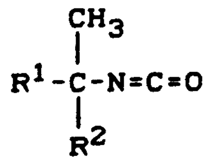 (II)

in welcher

$R^1$ und $R^2$ die oben angegebene Bedeutung haben,

mit elementarem Chlor gegebenenfalls unter Belichtung oder in Gegenwart von Katalysatoren in einer geeigneten Apparatur umsetzt.

**2.** Verfahren gemäß Anspruch 1, dadurch gekennzeichnet, daß $R^1$ für Methyl, Ethyl, n- oder iso-Propyl, n-, iso-, sec,-, tert.-Butyl und deren monohalogenierte Derivate steht und $R^2$ für Methyl, Ethyl, n- oder iso-Propyl, n-, iso-, sec.-oder tert.-Butyl und deren monohalogenierte Derivate oder für gegebenenfalls einfach bis dreifach, gleich oder verschieden durch Fluor, Chlor, Brom und/oder Trifluormethyl substituiertes Phenyl steht.

**3.** Verfahren gemäß Anspruch 1, dadurch gekennzeichnet, daß $R^1$ für Methyl oder Ethyl und $R^2$ für Methyl, Ethyl oder Phenyl stehen.

**4.** Verfahren gemäß Anspruch 1, dadurch gekennzeichnet, daß man Peroxide oder Azoverbindungen als Katalysatoren verwendet.

**5.** Verfahren gemäß Anspruch 1, dadurch gekennzeichnet, daß man die Umsetzung als Sumpfhalogenierung durchführt.

**6.** Verfahren gemäß Anspruch 5, dadurch gekennzeichnet, daß man die Reaktion bei einem Umsatz von 40-80 % unterbricht.

**7.** Verfahren gemäß Anspruch 1, dadurch gekennzeichnet, daß man die Reaktion als kontinuierliche Halogenierung durchführt.

**8.** Verfahren gemäß Anspruch 7, dadurch gekennzeichnet, daß man die Chlorierung bis zu einem Umsatz zwischen 0,1 und 30 % des durch die Chlorierungszone laufenden tert.-Alkylisocyanats durchführt.

Europäisches Patentamt

EUROPÄISCHER RECHERCHENBERICHT

Nummer der Anmeldung

EP 92 10 5526

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | EP-A-0 025 907 (BASF)<br>* Seite 4, Zeile 12 - Zeile 20; Anspruch 1; Beispiele 1,3,4,5 *<br>--- | 1-3,5-8 | C07C263/16 |
| A | US-A-3 468 923 (K KOENING ET AL)<br>* Spalte 2, Zeile 29 - Spalte 3, Zeile 7; Beispiele 1,3 *<br>--- | 1-8 | |
| A | US-A-3 535 360 (H HOLTSCHMIDT ET AL)<br>* Ansprüche 1-3 *<br>--- | 1-3,5-8 | |
| A | DE-A-1 418 666 (BASF)<br>* Seite 1 - Seite 5, Zeile 11; Beispiele 1,2,3 *<br>--- | 1-8 | |
| D,A | FR-A-2 107 796 (BASF)<br>* Anspruch 1; Beispiel 5 *<br>& DE-A-2 045 906<br>----- | 1-8 | |

| RECHERCHIERTE SACHGEBIETE (Int. Cl.5) |
|---|
| C07C |

Der vorliegende Recherchenbericht wurde für alle Patentansprüche erstellt

| Recherchenort | Abschlußdatum der Recherche | Prüfer |
|---|---|---|
| DEN HAAG | 08 JULI 1992 | HEYWOOD C.J. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)